Europäisches Patentamt

European Patent Office

Office européen des brevets

(19)

(11) EP 0 976 713 A1

(12) **EUROPEAN PATENT APPLICATION**

(43) Date of publication:
**02.02.2000 Bulletin 2000/05**

(51) Int Cl.7: **C07C 67/055**, C07C 69/145,
B01J 23/58, B01J 37/02

(21) Application number: **98306116.9**

(22) Date of filing: **31.07.1998**

(84) Designated Contracting States:
**AT BE CH CY DE DK ES FI FR GB GR IE IT LI LU MC NL PT SE**
Designated Extension States:
**AL LT LV MK RO SI**

(71) Applicant: **Dairen Chemical Corporation
Taipei (TW)**

(72) Inventors:
• **Chen, Shien-Chang
Taipei (TW)**
• **Lin, Fu-Shen
Kaohsiung (TW)**
• **Jong, Yuh-Lih
Kaohsiung (TW)**
• **Jang, Pi-Fwu
Kaohsiung (TW)**

(74) Representative: **Jones, Helen Marjorie Meredith
Gill Jennings & Every,
Broadgate House,
7 Eldon Street
London EC2M 7LH (GB)**

(54) **Preparation process of catalyst for producing alkenyl acetates and catalyst prepared by this process**

(57) This invention relates to a preparation process of a catalyst which comprises a noble metal, a metal for assisting catalysis, and an alkali or alkali earth metal compound, all of which are supported on the outer layer of a supporting materia. The preparation process comprises impregnating the supporting material with a solution containing an oxidative state noble metal as the main catalyst and an oxidative state metal for assisting catalysis, reducing the oxidative state metals into the metallic state in vapour phase with a gaseous reducing agent under constant temperature, constant pressure and constant gas concentration, then impregnating the treated supporting material with a solution of an alkali or alkali earth metal compound. The metal components-supporting catalyst prepared by the process according to the present invention has a high surface area and exhibits high catalytic activity, so that the catalytic ability and life of this heterophase catalyst can be improved. The catalyst prepared in the present invention is suitable for producing alkenyl acetates through reaction of olefins, acetic acid and oxygen in vapour phase.

EP 0 976 713 A1

**Description**

**Field of the Invention**

This invention relates to a process for the preparation of a catalyst which comprises a noble metal, a metal for assisting catalysis, and an alkali or alkali earth metal compound, all of which are supported on the outer layer of a supporting material. In a further embodiment this invention relates to a catalyst prepared according to the process of the present invention, which is suitable for producing alkenyl acetates through reaction of olefins, acetic acid and oxygen in vapour phase.

**Background of the Invention**

The industrial production of alkenyl acetates through reaction of olefins, oxygen and acetic acid in the vapour phase has been performed in the presence of a heterophase catalyst, which comprises a noble metal, a metal for assisting catalysis, and an alkali or alkali earth metal compound supported on the outer layer of a supporting material. This preparation process is known and the key point of this process, the metal components-supporting catalyst has been widely studied. Most of the research has been based on changing the kind or relative composition of the metal components. The basic structure of all catalysts used in the production of alkenyl acetates is essentially an egg-shell form produced by impregnating a supporting material with the metal components. The metal components are palladium metal, a metal for assisting preferred catalysis, and an alkali or alkali earth metal compound, wherein the metal for assisting catalysis is preferably gold and copper, and wherein the alkali or alkali earth metal compound is preferably a potassium compound as disclosed in US 3939199, EP 0361484 and USP 4668819.

US Patents 3725680 and 3743607 disclose catalysts employed to produce vinyl acetate, prepared by depositing palladium, gold and the like noble metals on the whole supporting material. US Patent 3,917,676 discloses a catalyst employed to produce propenyl acetate, prepared by depositing palladium, copper and the like noble metals on the whole supporting material. That is, the active noble metals are supported on the interiors and outer layers of a supporting material. A disadvantage of this process is that reactants employed in the catalytic process are not able to efficiently diffuse into inner regions of the supporting material, so that the interior active palladium and gold or copper metals are not able to be sufficiently utilised. In order to overcome this disadvantage the preparation process of the catalyst for producing alkenyl acetates, rather than depositing palladium metal and a metal for assisting catalysis on the whole supporting material, only a homogeneously impregnated layer was formed on the surface layer of a supporting material, so that a supported catalyst in the form of egg-shell was produced (US 4,087,622). The method for the preparation was that the active metallic materials were impregnated first, then these metal salts were precipitated through utilising alkali or alkali earth metal salts as disclosed in US 4,048,096, and US 3,775,342.

Further, it was known that when preparing the metal components-supporting catalyst, a special supporting substance was used. EP-A-0519435 describes how the supporting substance is cleaned with acids before the impregnation and treated with bases after the impregnation.

Since palladium and the metal for assisting catalysis were heterogeneous during the impregnation, their surface distribution on the supporting material was also heterogeneous. Research has been undertaken to investigate this problem (US-4,087,622, US 3,822,308 and British Patent 1521652). As a result, the preparation of the catalyst for producing alkenyl acetates has been essentially based on an egg-shell composition and structure formed by supporting palladium, a metal for assisting catalysis, and an alkali or alkali earth metal compound at the surface layer of a supporting material. The preparation process has generally comprised the following steps: (1) a supporting material is impregnated with an aqueous solution of soluble palladium ions and metal ions for assisting catalysis; (2) the impregnated supporting material is contacted with an alkali solution, so that the soluble palladium ions and metal ions for assisting catalysis are precipitated on the surface layer of the supporting material and formed into insoluble oxidative state palladium and metal for assisting catalysis; (3) the treated supporting material is washed with water to remove soluble ions produced during the precipitation; (4) the oxidative state palladium and metal for assisting catalysis supported on the treated supporting material are reduced to the metallic state; (5) the treated supporting material in (4) is impregnated with a solution of an alkali or alkali earth metal compound; and (6) the impregnated supporting material in (5) is dried. Among these steps, conventionally, the reduction step (4) is performed by a reduction process using liquid reducing agents in the liquid phase. However, since heterogeneity of the distribution of palladium and the metal for assisting catalysis on the surface of the supporting material still exists, the catalyst produced is not satisfactory.

In order to resolve the above problem, in the preparation process of the catalyst for producing alkenyl acetates according to the present invention, the conventional reducing process utilising liquid reducing agents in the liquid phase is changed into a reduction process utilising gaseous reducing agents in the vapour phase. As a result, the prepared metal components-supporting catalyst has a high surface area and exhibits high catalytic activity, so that the catalytic ability and life of this heterophase catalyst are improved.

**Summary of the Invention**

This invention relates to a preparation process of a catalyst which comprises a noble metal, a metal for assisting catalysis, and an alkali or alkali earth metal compound, all of which are supported on the outer layer of a supporting material. The preparation process comprises impregnating the supporting material with a solution containing an oxidative state noble metal as the main catalyst and an oxidative state metal for assisting catalysis, reducing the oxidative state metals into the metallic state in the vapour phase with a gaseous reducing agent under constant temperature, constant pressure and constant gas concentration, then impregnating the treated supporting material with a solution of an alkali or alkali earth metal compound. The metal components-supporting catalyst prepared by the process according to the present invention has a high surface area and exhibits high catalytic activity and longevity. The catalyst prepared in the present invention is suitable for producing alkenyl acetates through reaction of olefins, acetic acid and oxygen in the vapour phase.

**Detailed Description of the Invention**

This invention relates to a preparation process of a catalyst which comprises a noble metal, a metal for assisting catalysis, and an alkali earth metal compound, all of which are supported on the outer layer of a supporting material. Examples of a suitable supporting material are alumina, silica gel, silica, active carbon, silicon carbide, diatomaceous earth, pumice and the like, silica being preferable. The noble metal is palladium. Examples of the metal for assisting catalysis are gold, copper, molydenum, cadmium and magnesium, gold and copper being preferable. Examples of the alkali or alkali earth metal compounds are the hydroxides, acetates, nitrates and bicarbonates of potassium, sodium, cesium, magnesium, barium and the like. Potassium salts are preferable, and even more preferably, potassium acetate.

The present invention utilises gaseous reducing agents in the vapour phase to carry out the reduction of the oxidative state metals. After treatment with an alkali solution to convert the oxidative state noble metal and metal for assisting catalysis (which are supported on the supporting material after the impregnation) into hydroxides, these are reduced with suitable gaseous reducing agents in specific reductive conditions and converted into the metallic state.

The term "oxidative state" used herein according to the present invention means cationic metal ion, for example, oxidative state palladium means $Pd^{2+}$.

In the preparation process according to the present invention, after the metal components are supported on the surface layer of a supporting material by means of a conventional method, the heretofore nonreduced catalyst is placed in a reactor and a gaseous reducing agent is used under suitable reductive conditions in the vapour phase to reduce the oxidative state metals into free metals. Examples of gaseous reducing agents used in the present invention are hydrogen and ethylene, preferably hydrogen. In the reduction process, it is preferable to dilute the gaseous reducing agent with inert gas (such as nitrogen gas). The volume percentage of the reducing agent after dilution is in the range of 0.05 to 75%, and preferably, 5 to 30%. The amount of the reducing agent used depends on the amounts of the noble metal and the metal for assisting catalysis so that the equivalent thereof usually should be at least 1 to 1.5 times the equivalents required to reduce the catalyst; if necessary, more reducing agents can be used. The reduction temperature is usually 100 to 300°C, preferably 150 to 250°C. The reducing pressure is usually 0 to 5 kg/cm$^2$ (gauge pressure), preferably 1.5 to 3.5 kg/cm$^2$ (gauge pressure).

After the reduction process, the treated catalyst is washed with deionised water until the chloride ions are completely removed. After drying it is impregnated with an aqueous solution containing an alkali or alkali earth metal compound. Finally, the catalyst is dried at a temperature of 80 to 150°C until the water content is 0 to 6% by weight, preferably 1 to 3% by weight.

The metal components-supporting catalyst prepared in the present invention is suitable for producing alkenyl acetates through reaction of olefins, acetic acid and oxygen in vapour phase. The olefinic compounds include ethylene, propylen, isobuylene and the like. For example, the catalyst prepared in the present invention can be used in the presence of ethylene, acetic acid and oxygen in vapour phase to produce vinyl acetate. The catalyst employed in the production process comprises palladium, gold and an alkali or alkali earth metal compound, preferably a potassium compound. Also, the catalyst prepared in the present invention may be used in the presence of propylene, acetic acid and oxygen in the vapour phase to produce propenyl acetate. The catalyst employed in this production process comprises palladium, copper (barium and lead can further be added), and an alkali or alkali earth metal compound, preferably a potassium compound.

A quantity of the above prepared catalyst, for producing alkenyl acetates, is placed in a reaction tube with an inner diameter of 20mm and a length of 2.0m. Under a specific pressure at the entrance of the reaction tube, the reactant gases are introduced into the tube at a reaction temperature set according to the activity of the catalyst. These reactant gases comprise 30 to 45 volume % of olefin, 30 to 50 volume % of nitrogen gas, 5 to 15 volume % of acetic acid and 3 to 7 volume % of oxygen. Analysis of the yield of alkenyl acetate in the composition at the exit of the tube is performed in a definite time.

Generally, the selection of a catalyst in the industry is based on the catalytic activity. The catalytic activity can be calculated according to the following formula:

The activity of a catalyst:

$$STY(\text{space time yield})=\frac{\text{weight of alkenyl acetates produced (g)}}{\text{volume(1) of catalyst x sampling time (hr)}}$$

The selectivity of a catalyst:

$$C_2 \text{ selectivity}=\frac{\text{moles of vinyl acetate produced}}{\text{moles of vinyl acetate produced}+1/2 \text{ moles of } CO_2 \text{ produced}}$$

$$C_3 \text{ selectivity}=\frac{\text{moles of propenyl acetate produced}}{\text{moles of propenyl acetate produced}+1/3 \text{ moles of } CO_2 \text{ produced}}$$

As a consequence of the greater surface area of metals in the catalyst prepared according to the present invention compared to the catalysts prepared by the prior art methods the efficiency of the catalyst in the present invention is higher. Analysis of the catalytic activity of the catalyst of the present invention when used to produce alkenyl acetates confirms that it not only provides higher catalytic activity, but has an extended life. Compared to the conventional catalyst, the catalyst of the present invention is able to yield more alkenyl acetates per unit volume of the reactor and per unit time when the conditions of the synthetic reaction (such as pressure, temperature, oxygen concentration) remain constant. Moreover, if the productive yield remains constant, then the reacting temperature can be decreased. Therefore, since the selectivity of the reaction is variable under the same output, the cost of the starting materials is lower. The amount of carbon dioxide by-product is also reduced as are the products lost during the exclusion of carbon dioxide.

The invention will be further described with reference to the following Examples and Comparative Examples, but the scope of the present invention is by no means limited.

## Example 1

The supporting material employed in this Example was a porous carrier of alumina/silica with an outer diameter of 5 mm and was available from SUD-CHEMIE AG. This supporting material had a surface area of 100 to 120 $m^2/g$, a pore volume of 0.7 to 0.9 ml/g and a volume density of 600 g/l. The metal component-supporting catalyst was prepared according to the following steps:

Step 1): An aqueous $Na_2PdCl_4$ solution with weight of 2.2 kg containing 15 weight % of palladium was added into an aqueous $HAuCl_4$ solution with weight of 0.5 lg containing 30 weight % of gold. The mixture was then diluted with deionised water until total volume was 37.2 1. 100 1 of alumina/silica carrier was placed in an impregnating tank with rotation rate of 24 turns per minute. The mixture was added rapidly in 10 minutes.

Step 2): Hot air was passed through to dry the carrier until the remained moisture was less than 4%. The temperature of the hot air was lower than 120°C.

Step 3): 28 weight % NaOH solution with weight of 160% the amount absorbed by the carrier (about 60 kg) was added to the dried catalyst. The impregnating time was over 20 hours. The originally soluble chloride state palladium and gold were converted into soluble hydroxides of palladium and gold.

Step 4): The impregnated catalyst carrier after drying was placed in a reduction reactor with temperature controlled at 165°C and pressure set at 2 $kg/cm^2$ (gauge pressure). The reducing gases were passed into the reactor with a flow rate of 15 cm/sec, wherein the composition of the reducing gases was hydrogen : nitrogen = 1 : 3. The hydroxide state palladium and gold were reduced to metallic state palladium and gold.

Step 5) : The above catalyst was washed to remove chloride ions with the amount of 15 to 16 litres of deionised water per litre of the catalyst until the catalyst was free of chloride ions.

Step 6): The catalyst carrier was dried as in step 2).

Step 7): An adequate amount of potassium acetate was added into the dried catalyst carrier, so that each litre of the catalyst contained 30g weight of potassium acetate.

Step 8): The catalyst carrier was dried as in step 2).

After the above steps, a catalyst contained 3.3 g/l of palladium, 1.5 g/l of gold and 30 g/l of potassium acetate was obtained, wherein all palladium and gold were well distributed on the shell of the carrier.

Nine hundred millilitres of the catalyst thus obtained was charged into a reaction tube with an inner diameter of 20 mm and a length of 2.0 m. Under a pressure of 8 kg/cm$^2$ (gauge pressure) at the entrance of the reactor, the reactant gaseous mixture was introduced into the reactor at a temperature of 140°C. The gaseous mixture comprised 41 volume % of ethylene, 43 volume % of nitrogen gas, 10 volume % of acetic acid and 6 volume % of oxygen. The composition at the exit was analysed in a definite time, the activity and the selectivity of the catalyst were calculated, and the surface area of metals was determined. The results are listed in Table 1.

When the activity and the selectivity of the catalyst were evaluated, the crude product at the exit of the reactor was cooled with chilled water, and the composition was analysed by Shimadzu Gas Chromatography. The flow rate of the gases was determined by Shinagawa Dry Gas Meter and the surface area of metals was determined according to ChemiSorp Method as in ASTM D3908.

### Example 2

A similar process to that of example 1 was carried out except that an aqueous $Na_2PdCl_4$ solution with weight of 2.2 kg containing 18 weight % of palladium and an aqueous $HAuCl_4$ solution with weight of 0.5 kg containing 36 weight % of gold were prepared. A catalyst was obtained that contained 4.0 g/l of palladium, 1.8 g/l of gold and 30 g/l of potassium acetate, wherein all palladium and gold were well distributed on the shell of the carrier.

This catalyst was evaluated by the same method as in Example 1,and the results are listed n Table 1.

### Example 3

A similar process to that of example 1 was carried out except that an aqueous $Na_2PdCl_4$ solution with weight of 2.2 kg containing 22.5 weight % of palladium and an aqueous $HAuCl_4$ solution with weight of 0.5 kg containing 45 weight % of gold were prepared. As well, a catalyst was obtained that contained 5.0 g/l of palladium, 2.25 g/l of gold and 30 g/l of potassium acetate, wherein all palladium and gold were well distributed on the shell of the carrier.

This catalyst was evaluated by the same methods as in Example 1, and the results are listed in Table 1.

### Example 4

A similar process to that of example 1 was carried out except that an aqueous $Na_2PdCl_4$ solution with weight of 2.2 kg containing 30 weight % of palladium and an aqueous $HAuCl_4$ solution with weight of 0.5 kg containing 60 weight % of gold were prepared. As well, a catalyst was obtained that contained 6.6 g/l of palladium, 3.0 g/l of gold and 30 g/l of potassium acetate, wherein all palladium and gold were well distributed on the shell of the carrier.

This catalyst was evaluated by the same methods as in Example 1, and the results are listed in Table 1.

### Comparative Example 1

A similar process to that of example 1 was carried out wherein the catalyst was prepared with an aqueous $Na_2PdCl_4$ solution with weight of 2.2 kg containing 15 weight % of palladium and an aqueous $HAuCl_4$ solution with weight of 0.5 kg containing 30 weight % of gold except that step 4) was altered as follows: the impregnated 5 mm porous carrier after step 3) was poured into 50 1 of an aqueous solution containing 5% $N_2H_4$ for 4 hours, so that the hydroxide state palladium and gold deposited on the carrier were reduced into metallic state palladium and gold. The same procedures thereafter as in Example 1 were followed, and the carrier was washed, potassium acetate was added and the catalyst carrier was dried.

This catalyst was evaluated by the same methods as in Example 1, and the results are listed in Table 1.

### Comparative Example 2

Catalyst preparation was carried out as in Comparative Example 1 except that an aqueous $Na_2PdCl_4$ solution with weight of 2.2 kg containing 18 weight % of palladium and an aqueous $HAuCl_4$ solution with weight of 0.5 kg containing 36 weight % of gold were prepared .

This catalyst was evaluated by the same methods as in Example 1, and the results are listed in Table 1.

### Comparative Example 3

Catalyst preparation was carried out as in Comparative Example 1 except that an aqueous $Na_2PdCl_4$ solution

with weight of 2.2 kg containing 22.5 weight % of palladium and an aqueous $HAuCl_4$ solution with weight of 0.5 kg containing 45 weight % of gold were prepared.

This catalyst was evaluated by the same methods as in Example 1, and the results are listed in Table 1.

## Comparative Example 4

Catalyst preparation was carried out as in Comparative Example 1 except that an aqueous $Na_2PdCl_4$ solution with weight of 2.2 kg containing 30 weight % of palladium and an aqueous $HAuCl_4$ solution with weight of 0.5 kg containing 60 weight % of gold were prepared.

This catalyst was evaluated by the same methods as in Example 1, and the results are listed in Table 1.

## Example 5

Catalyst preparation was carried out as in Comparative Example 1 except that $Na_2PdCl_4$ solution with weight of 2.2 kg containing 15 weight % of palladium and an aqueous $CuCl_2$ solution with weight of 0.5 kg containing 14.6 weight % of copper were prepared.

Subsequent to the above steps, a catalyst was obtained that contained 3.3 g/l of palladium, 0.34 g/l of copper and 30 g/l of potassium acetate, wherein all palladium and copper were well distributed on the shell of the carrier.

Six hundred millilitres of thus obtained catalyst was charged into a reaction tube with an inner diameter of 20 mm and a length of 2.0 m. Under a pressure of 7.0 kg/cm$^2$ (gauge pressure) at the entrance of the reactor, the reactant gaseous mixture was introduced into the reactor at a temperature of 148°C. The gaseous mixture was of 29 volume % of propylene, 44 volume % of nitrogen gas, 6.7 volume % of acetic acid 13.5 volume % of steam and 6.5 volume % of oxygen. According to the same methods as in Example 1, while the composition at the exit was analysed in a definite time, the activity and the selectivity of the catalyst were calculated, and the surface area of metals was determined. The results are listed in Table 1.

## Example 6

Catalyst preparation was carried out as in Example 1 except that an aqueous $Na_2PdCl_4$ solution with weight of 2.2 kg containing 15 weight % of palladium and an aqueous $CuCl_2$ solution with weight of 0.5 kg containing 6.0 weight % of copper were prepared.

This catalyst was evaluated by the same methods as in Example 5, and the results are listed in Table 1.

## Example 7

Catalyst preparation was carried out as in Example 1 except that an aqueous $Na_2PdCl_4$ solution with weight of 2.2 kg containing 22.5 weight % of palladium and an aqueous $CuCl_2$ solution with weight of 0.5 kg containing 14.6 weight % of copper were prepared.

This catalyst was evaluated by the same methods as in Example 5, and the results are listed in Table 1.

## Comparative Example 5

Catalyst preparation was carried out as in Comparative Example 1 except that an aqueous $Na_2PdCl_4$ solution with weight of 2.2 kg containing 15 weight % of palladium and an aqueous $CuCl_2$ solution with weight of 0.5 kg containing 14.6 weight % of copper were prepared.

This catalyst was evaluated by the same methods as in Example 5, and the results are listed in Table 1.

## Comparative Example 6

Catalyst preparation was carried out as in Comparative Example 1 except that an aqueous $Na_2PlCl_4$ solution with weight of 2.2 kg containing 15 weight % of palladium and an aqueous $CuCl_2$ solution with weight of 0.5 kg containing 6.0 weight % of copper were prepared.

This catalyst was evaluated by the same methods as in Example 5, and the results are listed in Table 1.

## Comparative Example 7

Catalyst preparation was carried out as in Comparative Example 1 except that an aqueous $Na_2PdCl_4$ solution with weight of 2.2 kg containing 22.5 weight % of palladium and an aqueous $CuCl_2$ solution with weight of 0.5 kg containing 14.6 weight % of copper were prepared.

This catalyst was evaluated by the same methods as in Example 5, and the results are listed in Table 1.

**Comparative Example 8**

This is a repetition of Example 1 except that instead of hydrogen, ethylene was used as the gaseous reducing agent in the reducing process.

This catalyst was evaluated by the same methods as in Example 1, and the results are listed in Table 1.

|  | Activity (g/l/hr) | Selectivity (%) | Surface Area of Metals (m$^2$/g.metal) |
|---|---|---|---|
| Example 1 | 401 | 95.4 | 138 |
| Example 2 | 456 | 95.7 | 143 |
| Example 3 | 632 | 94.8 | 123 |
| Example 4 | 785 | 95.4 | 117 |
| Comparative Example 1 | 327 | 95.5 | 123 |
| Comparative Example 2 | 380 | 95.6 | 122 |
| Comparative Example 3 | 440 | 94.1 | 102 |
| Comparative Example 4 | 537 | 92.6 | 101 |
| Example 5 | 609 | 97.5 | 120 |
| Example 6 | 554 | 97.0 | 127 |
| Example 7 | 689 | 97.3 | 125 |
| Comparative Example 5 | 547 | 96.4 | 105 |
| Comparative Example 6 | 497 | 96.0 | 109 |
| Comparative Example 7 | 612 | 96.7 | 109 |
| Comparative Example 8 | 306 | 96.2 | 101 |

**Claims**

1.  A process for the preparation of a catalyst, comprising the steps of (a) impregnation of a catalyst supporting material surface with a solution containing an oxidative state noble metal and an oxidative state metal for assisting catalysis, (b) reducing the metals from the oxidative state into the metallic state in the vapour phase with gaseous reducing agent; (c) impregnating the reduced catalyst with a solution of alkali or alkali earth metal compound, and (d) drying the catalyst.

2.  The process according to claim 1, wherein the temperature in the range of 100 to 300°C

3.  The process according to claim 1 or claim 2, wherein the pressure in the range of 0 to 5 kg/cm$^2$ (gauge pressure);

4.  The process according to any preceding claim, wherein said noble metal is palladium.

5.  The process according to any preceding claim, wherein said metals for assisting catalysis are gold, copper, molybdenum, cadmium and magnesium.

6.  The process according to any preceding claim, wherein said alkali or alkali earth metal compound are the hydroxides, acetates, nitrates and bicarbonate of potassium, sodium, cesium, magnesium and barium.

7.  The process according to any preceding claim, wherein said catalyst supporting material are alumina, silica gel, silica, active carbon, silicon carbide, diatomaceous earth and pumice.

8.  The process according to any preceding claim, wherein said gaseous reducing agent is hydrogen.

9. A catalyst obtainable by the process according to any preceding claim.

10. The use of a catalyst as defined in claim 9 for the production of alkenyl acetates by reaction with olefins, acetic acid and oxygen.

11. The use according to claim 10, wherein said olefins are ethylene, propylene and isobutylene.

12. The use according to claim 9, wherein said alkenyl acetate is vinyl acetate or propenyl acetate.

European Patent
Office

**EUROPEAN SEARCH REPORT**

Application Number

EP 98 30 6116

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO 98 18553 A (MILLENIUM PETROCHEMICAL) 7 May 1998 ESPECIALLY PAGES 10-13, 17, 13 | 1,2,4-12 | C07C67/055 C07C69/145 B01J23/58 B01J37/02 |
| X | US 4 321 409 A (Y. YOSHIDA ET AL.) 23 March 1982 ESPECIALLY COLUMN 3, LINE 27 TO COLUMN 4, LINE29 AND COLUMNS 5-6, EXAMPLES 1 TO 17 | 1,2,4-10 | |
| X | US 5 691 267 A (I. NICOLAU ET AL.) 25 November 1997 ESPECIALLY COLUMN 7, LINES 14-37 AND COLUMN 9, LINES 13-50 | 1,2,4-12 | |
| A | US 5 225 388 A (F. WUNDER ET AL) 6 July 1993 * column 4, line 64 - column 5, line 17 * | 2 | |

TECHNICAL FIELDS
SEARCHED    (Int.Cl.6)

C07C
B01J

The present search report has been drawn up for all claims

| Place of search | Date of completion of the search | Examiner |
|---|---|---|
| THE HAGUE | 22 December 1998 | Devisme, F |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

**ANNEX TO THE EUROPEAN SEARCH REPORT
ON EUROPEAN PATENT APPLICATION NO.**     EP 98 30 6116

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-1998

| Patent document cited in search report | | Publication date | Patent family member(s) | | Publication date |
|---|---|---|---|---|---|
| WO 9818553 | A | 07-05-1998 | AU | 5102998 A | 22-05-1998 |
| US 4321409 | A | 23-03-1982 | JP | 1249082 C | 25-01-1985 |
|  |  |  | JP | 55098138 A | 25-07-1980 |
|  |  |  | JP | 59017098 B | 19-04-1984 |
| US 5691267 | A | 25-11-1997 | AU | 2724097 A | 07-11-1997 |
|  |  |  | WO | 9738790 A | 23-10-1997 |
| US 5225388 | A | 06-07-1993 | DE | 3940125 A | 06-06-1991 |
|  |  |  | AU | 629072 B | 24-09-1992 |
|  |  |  | AU | 6771890 A | 13-06-1991 |
|  |  |  | CA | 2031429 A | 06-06-1991 |
|  |  |  | DE | 59007054 D | 13-10-1994 |
|  |  |  | EP | 0431478 A | 12-06-1991 |
|  |  |  | ES | 2060905 T | 01-12-1994 |
|  |  |  | JP | 4108759 A | 09-04-1992 |
|  |  |  | MX | 173115 B | 01-02-1994 |

EPO FORM P0459

For more details about this annex : see Official Journal of the European Patent Office, No. 12/82